# EUROPÄISCHE PATENTANMELDUNG

(11) **EP 0 705 612 A1**
(43) Veröffentlichungstag der Anmeldung: **10.04.1996**
(21) Anmeldenummer: 95114929.3
(22) Anmeldetag: 22.09.1995
(51) Int. Cl.: A61M 5/172, G06F 3/023

(54) **Medizinisches Gerät mit eine Dosiervorrichtung**

(30) Priorität: 08.10.1994 DE 4436014
(71) Anmelder: DRÄGERWERK AKTIENGESELLSCHAFT, D-23542 Lübeck (DE)
(72) Erfinder: Hölscher, Uvo, Dr., D-23617 Stockelsdorf (DE)

(57) **Zusammenfassung**

Ein medizinisches Gerät (1) mit einer Dosiervorrichtung (2, 3) für ein fluides Medium (4, 5), mit einer Recheneinheit (19) zur Steuerung der Dosiervorrichtung (2, 3) nach einzelnen Vorgabegrößen, einem Anzeigebildschirm (12) zur Darstellung von möglichen Vorgabegrößen innerhalb von Menüstrukturen, einem Drehknopf (13) zur Auswahl einer Vorgabegröße, einem druckbetätigten Quittierschalter (15) zur Übernahme der ausgewählten Vorgabegrößen als ein Einstellwert für eine der Dosiervorrichtungen (2), und mit einem weiteren Schalter (16) soll hinsichtlich einfacherer Korrekturmöglichkeit einer irrtümlich übernommenen Vorgabegröße verbessert werden.
Zur Lösung der Aufgabe ist vorgesehen, daß der weitere Schalter als ein durch den Drehknopf (13) betätigter Zug- oder Schiebeschalter (16) ausgeführt ist, durch welchen ein die Übernahme der Vorgabegröße als Einstellwert aufhebendes Signal S1 an die Recheneinheit (19) und/oder die Dosiervorrichtung (2) geschaltet ist.

## Beschreibung

Die Erfindung betrifft ein medizinisches Gerät mit einer Dosiervorrichtung für ein fluides Medium, mit einer Recheneinheit zur Steuerung der Dosiervorrichtung nach einzelnen Vorgabegrößen, einem Anzeigebildschirm zur Darstellung von möglichen Vorgabegrößen innerhalb von Menüstrukturen, einem Drehknopf zur Auswahl einer Vorgabegröße, einem druckbetätigten Quittierschalter zur Übernahme der ausgewählten Vorgabegröße als ein Einstellwert für die Dosiervorrichtung und mit einem weiteren Schalter.

Ein Beatmungsgerät mit einer Misch- und Dosiervorrichtung für medizinische Gase zur Beatmung eines Patienten ist aus der US 5,237,987 bekannt geworden. Das bekannte Beamtungsgerät besitzt eine Steuereinrichtung, mittels derer der Benutzer die Beatmungsform, den Beatmungsdruck, die Gasartzusammensetzung und den Beatmungsgasfluß einstellen und überwachen kann. Bestandteil der Steuereinrichtung ist ein Anzeigebildschirm, über den mittels Menüstrukturen eingestellte Parameter als Vorgabegrößen dem Benutzer angezeigt werden. Sollen Parameter verändert werden, kann der Benutzer zunächst mittels eines zentral angeordneten Drehknopfes den zu ändernden Parameter innerhalb einer der Menüstrukturen anwählen, durch Druck auf einen Quitterschalter den zu ändernden Parameter selektieren, innerhalb eines Menüfensters den Parameter dann zahlenmäßig variieren, und den neuen Parameter durch Druck auf den Quittierschalter als einen neuen Einstellwert für das Beatmungsgerät in die Steuereinrichtung übernehmen. Danach kann ein anderer Parameter aus der Menüstruktur selektiert und bei Bedarf geändert werden. Mittels eines weiteren Schalters kann zwischen einer die Parameter anzeigenden und einer die Parameter ändernden Menüstruktur umgeschaltet werden.

Nachteilig bei dem bekannten Beatmungsgerät ist, daß bei der zahlenmäßigen Veränderung eines selektierten Parameters und versehentlicher Betätigung des Quittierschalters, eine Korrektur des irrtümlich übernommenen Parameters nicht unmittelbar möglich ist. Vielmehr muß der falsch eingestellte Parameter zunächst wieder selektiert werden, um danach zahlenmäßig auf den gewünschten Wert eingestellt werden zu können. Dieses erschwert, insbesondere in Streßsituationen, die Handhabung.

Der Erfindung liegt die Aufgabe zugrunde, ein medizinisches Gerät der genannten Art derart zu verbessern, daß eine einfache Korrektur einer irrtümlich übernommenen Vorgabegröße als Einstellwert möglich ist.

Die Lösung der Aufgabe erfolgt dadurch, daß der weitere Schalter als ein durch den Drehknopf betätigter Zug- oder Schiebeschalter ausgeführt ist, durch welchen ein die Übernahme der Vorgabegröße als Einstellwert aufhebendes Signal an die Recheneinheit und/oder die Dosiervorrichtung geschaltet ist.

Der Vorteil der Erfindung besteht im wesentlichen darin, daß durch Zug oder seitliches Verschieben des Drehknopfes die Übernahme der zuvor geänderten, neuen Vorgabegröße unmittelbar aufgehoben werden kann, ohne daß weitere Bedienungsschritte in Form einer nochmaligen Selektion der betreffenden Vorgabegröße zwischengeschaltet sind. Die Art und Weise der Betätigung des Drehknopfes, d.h. durch Zug oder durch seitliches Verschieben, gewährleistet, daß das die Übernahme aufhebende Signal nur bewußt durch den Benutzer geschaltet werden kann.

In einer zweckmäßigen Ausgestaltung der Erfindung ist vorgesehen, daß durch das die Übernahme aufhebende Signal gleichzeitig die vor der Übernahme der Vorgabegröße angezeigte Menüstruktur wieder an den Anzeigebildschirm geschaltet ist. Auf diese Weise ist eine umittelbare Neueinstellung der Vorgabegröße möglich.

In einer vorteilhaften Ausgestaltung der Erfindung ist ein medizinisches Gerät mit einer Recheneinheit zur Aufnahme von Statusgrößen, einem Anzeigebildschirm zur Darstellung von möglichen Vorgabegrößen für die Statusgrößen innerhalb von Menüstrukturen, einem Drehknopf zur Auswahl einer Vorgabegröße, einem druckbetätigten Quitierschalter zur Übernahme der ausgewählten Vorgabegröße und einem weiteren Schalter in der Weise ausgeführt, daß der weitere Schalter ein durch den Drehknopf betätigter Zug- oder Schiebeschalter ist, durch welchen ein die Übernahme der Vorgabegröße aufhebendes Signal S1 an die Recheneinheit geschaltet ist.

Die Statusgrößen können von Sensoren registrierte Meßgrößen sein, z.B. die Dosierrate einer Medikamenten-Lösung, welche sich aus der Verschiebung eines durch einen Linearantrieb betätigten Kolbens ergibt. Der Linearantrieb kann hierzu mit einer die Verschiebung des Kolbens messenden Wegmeßeinrichtung versehen sein. Die Vorgabegrößen sind Grenzwerte für die Dosierrate , die nicht unterschritten oder überschritten werden dürfen. Alternative Meßgrößen zur Dosierrate sind beispielsweise für Anwendungsfälle im Bereich der Beatmung, das Tidalvolumen, der Beatmungsdruck, die Atemfrequenz, die Herzfrequenz, die Sauerstoff-Konzentration und die Anästhesiemittel-Konzentration. Die Vorgabewerte für diese alternativen Meßgrößen sind die einzuhaltenden Grenzwerte dieser Meßgrößen.

Ein Ausführungsbeispiel der Erfindung ist in der Zeichnung dargestellt und im folgenden näher erläutert.

Es zeigen:
- Figur 1: den schematischen Aufbau eines medizinischen Gerätes mit einer Dosierpumpe,
- Figur 2a: eine erste Menüstruktur mit einem ersten Menüfenster,
- Figur 2b: eine zweite Menüstruktur mit einem zweiten Menüfenster.

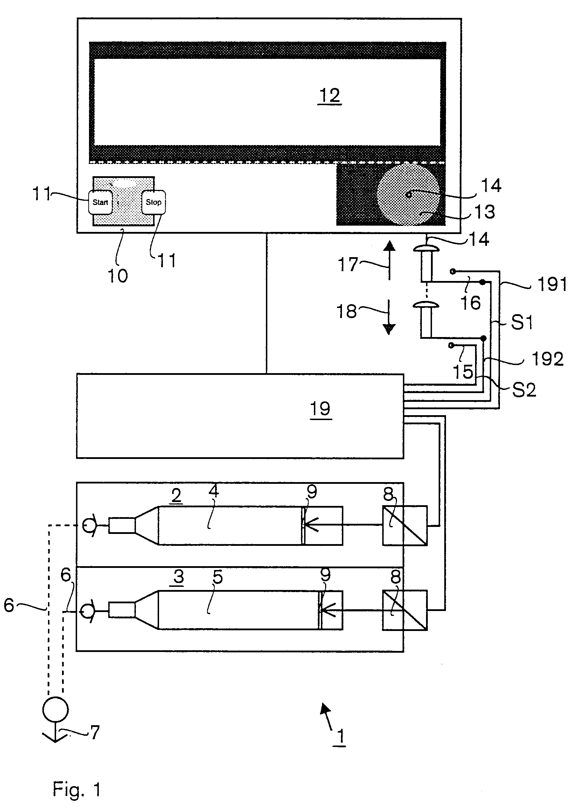

Figur 1 zeigt schematisch den Aufbau eines medizinischen Gerätes (1) mit zwei Dosierpumpen (2, 3) zur Dosierung von Medikamenten-Lösungen (4, 5) über Fluidleitungen (6) in eine Sammelleitung (7). Die Dosierpumpen (2, 3) sind als Kolbenpumpen ausgeführt, und die Lösungen (4, 5) werden jeweils mittels eines durch einen Linearantrieb (8) betätigten Kolbens (3) in die zugehörige Fluidleitung (6) gefördert. Die Linearantriebe (8) enthalten jeweils eine in der Figur 1 nicht dargestellte Wegmeßeinrichtung, mit der die axiale Verschiebung der Kolben (3) erfaßt werden kann, wobei aus den Verschiebungen in einer Recheneinheit (19) die Dosierraten der Medikamenten-Lösungen (4, 5) bestimmt werden.
Die Einstellung der Vorgabegrößen für die Dosierpumpen (2, 3) erfolgt über eine Bedienoberfläche (10), auf der verschiedene Schalter (11), ein Anzeigebildschirm (12) und ein Drehknopf (13) zur Einstellung von Vorgabewerten angeordnet sind. Der Drehknopf (13) ist um eine zentrisch angeordnete Achse (14) drehbar, welche mit einem Quittierschalter (15) als Druckschalter und einem Zugschalter (16) in Verbindung steht. Der besseren Übersicht wegen ist der hinter der Bedienoberfläche (10) liegende Teil der Achse (14), in der Figur 1 gestrichelt gezeichnet, in die Zeichenebene geklappt worden. Bei Zug an dem Drehknopf (13) in Richtung des ersten Pfeils (17) schaltet der Zugschalter (16) in Schließstellung, und bei Druck auf den Drehknopf (13) in Richtung des zweiten Pfeils (18), wird der Quittierschalter (15) geschlossen. Die Steuerung der Dosierung erfolgt mittels der zentralen Recheneinheit (19) mit einem in der Figur 1 nicht dargestellten Mikroprozessor innerhalb der Recheneinheit (19). Über eine erste Leitung (191) ist ein erstes Signal S1 mittels des Zugschalters (16) an die Recheneinheit (19) schaltbar, und über eine zweite Leitung (192) wird mit dem Quittierschalter (15) ein zweites Signal S2 an die Recheneinheit (19) gelegt. Mit den Schaltern (11) auf der Bedienoberfläche (10) können die Dosierpumpen (2, 3) gestartet bzw. gestoppt werden.

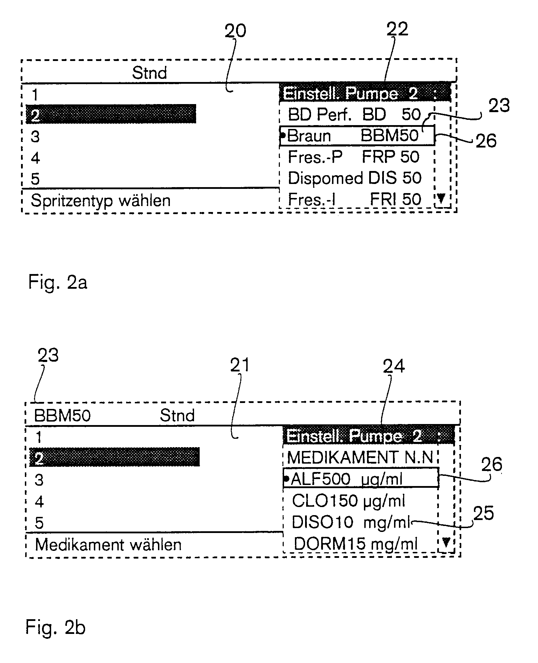

In den Figuren 2a und 2b sind Beispiele für mit dem Anzeigebildschirm (12) darstellbare Menüstrukturen (20, 21) gezeigt. Eine erste Menüstruktur (20), zur Auswahl einer speziellen Bauform der Dosierpumpen (2, 3), enthält in einem ersten Menüfenster (22) verschiedene Spritzen-Vorgabegrößen (23). Eine zweite Menüstruktur (21) besitzt innerhalb eines zweiten Menüfensters (24) eine Medikamenten-Bibliothek mit Medikamenten-Vorgabewerten (25) und Dosierraten für die Medikamente.

Die Arbeitsweise des erfindungsgemäßen medizinischen Gerätes (1) ist folgendermaßen:
Mittels eines durch den Drehknopf (13) verschiebbaren Cursors (26) wird innerhalb des ersten Menüfensters (22), Fig. 2a, der ersten Menüstruktur (20) für die Dosierpumpe (2) als Spritzen- Vorgabegröße (23) der Typ BBM50 ausgewählt. Durch Betätigung des Quittierschalters (15) wird diese Vorgabegröße (23) dann in die Recheneinheit (19) übernommen. Als Steuersignal erhält die Recheneinheit (19) hierzu das zweite Signal S2 über den Quittierschalter (15). Gleichzeitig wird mittels des zweiten Signals S2 die zweite Menüstruktur (21), Fig. 2b, mit dem zweiten Menüfenster (24) auf den Anzeigebildschirm geschaltet. Mittels des Drehknopfes (13) und des Cursors (26) kann eine Medikamenten-Vorgabegröße (25) ausgewählt werden. Die zuvor übernommene Spritzen-Vorgabegröße (23) erscheint als Einstellwert in der linken Ecke der zweiten Menüstruktur (21). Ist die Spritzen-Vorgabegröße (23) versehentlich übernommen worden, ist ein Rücksprung in die vorherige, erste Menüstruktur (20) durch Zug an dem Drehknopf (13) möglich, wodurch der Zugschalter (16) in Schließstellung schaltet und durch das an die Recheneinheit (19) geschaltete erste Signal S1, der Rücksprung in die erste Menüstruktur (20) ausgeführt und gleichzeitig die Übernahme der Spritzen-Vorgabegröße (23) in die Recheneinheit (19) aufgehoben wird. Mit dem Drehknopf (13) kann dann eine neue Spritzen-Vorgabegröße (23) ausgewählt und durch Betätigung des Quittierschalters (15) in die Recheneinheit übernommen werden.

In einem weiteren, in den Figuren nicht dargestellten Menüfenster, können mit dem Cursor (26) als Vorgabewerte in den Figuren ebenfalls nicht dargestellte obere- und untere Grenzwerte für die Dosierrate ausgewählt und durch Druck auf den Quittierschalter (15) in die Recheneinheit (19) übernommen werden. Ein übernommener Grenzwert kann durch Zug an dem Drehknopf (13) wieder aufgehoben werden. Anschließend ist die Auswahl eines neuen Grenzwertes möglich.

## Patentansprüche

1. Medizinisches Gerät (1) mit einer Dosiervorrichtung (2, 3) für ein fluides Medium (4, 5), mit einer Recheneinheit (19) zur Steuerung der Dosiervorrichtung (2, 3) nach einzelnen Vorgabegrößen (23, 25), einem Anzeigebildschirm (12) zur Darstellung von möglichen Vorgabegrößen (23, 25) innerhalb von Menüstrukturen (20, 21), einem Drehknopf (13) zur Auswahl einer Vorgabegröße (23), einem druckbetätigten Quittierschalter (15) zur Übernahme der ausgewählten Vorgabegröße (23) als ein Einstellwert für eine Dosiervorrichtung (2) und mit einem weiteren Schalter (16), dadurch gekennzeichnet, daß der weitere Schalter als ein durch den Drehknopf (13) betätigter Zug- oder Schiebeschalter (16) ausgeführt ist, durch welchen ein die Übernahme der Vorgabegröße (23) als Einstellwert aufhebendes Signal S1 an die Recheneinheit (19) und/oder die Dosiervorrichtung (2) geschaltet ist.

2. Medizinisches Gerät nach Anspruch 1, dadurch gekennzeichnet, daß durch das Signal (S1) die vor der Übernahme der Vorgabegröße (23) angezeigte Menüstruktur (20) an den Anzeigebildschirm (12) geschaltet ist.

3. Medizinisches Gerät mit einer Recheneinheit (19) zur Aufnahme von Statusgrößen, einem Anzeigebildschirm (12) zur Darstellung von möglichen Vorgabegrößen für die Statusgrößen innerhalb von Menüstrukturen, einem Drehkopf (13) zur Auswahl einer Vorgabegröße, einem druckbetätigten Quittierschalter (15) zur Übernahme der ausgewählten Vorgabegröße und mit einem weiteren Schalter (16) dadurch gekennzeichnet, daß der weitere Schalter als ein durch den Drehknopf (13) betätigter Zug- oder Schiebeschalter (16) ausgeführt ist, durch welchen ein die Übernahme der Vorgabegröße aufhebendes Signal S1 an die Recheneinheit (19) geschaltet ist.

4. Medizinisches Gerät nach Anspruch 3, dadurch gekennzeichnet, daß durch das Signal S1 die vor der Übernahme der Vorgabegröße angezeigte Menüstruktur an den Anzeigebildschirm (12) geschaltet ist.
